# EUROPÄISCHE PATENTSCHRIFT

(11) **EP 1 654 210 B1**
(45) Veröffentlichungstag und Bekanntmachung des Hinweises auf die Patenterteilung: **30.07.2008**
(21) Anmeldenummer: 04737409.5
(22) Anmeldetag: 09.08.2004
(51) Int. Cl.: C07C 39/21, C07C 43/215, C07C 43/23, A61K 31/09, A61K 31/05, A61P 35/00

(54) **STILBEN-DERIVATE UND DEREN VERWENDUNG IN ARZNEIMITTELN**
STILBENE DERIVATIVES AND THEIR USE IN MEDICAMENTS
DERIVES DE STILBENE, ET LEUR UTILISATION POUR PRODUIRE DES MEDICAMENTS

(30) Priorität: 14.08.2003 AT 12852003
(43) Veröffentlichungstag der Anmeldung: 10.05.2006
(73) Patentinhaber: Szekeres, Thomas, 1010 Wien (AT)
(72) Erfinder: SZEKERES, Thomas, A-1010 Wien (AT); HANDLER, Norbert, A-1060 Wien (AT); JÄGER, Walter, A-3021 Pressbaum (AT); MURIAS, Arkadiusz, Marek, 60-694 Poznan (PL); ERKER, Thomas, A-1140 Wien (AT)
(74) Vertreter: Dungler, Karin
(86) Internationale Anmeldenummer: PCT/AT2004/000279
(87) Internationale Veröffentlichungsnummer: WO 2005/016860

(56) Entgegenhaltungen:
- WO-A-02/50007
- WO-A-20/04000302
- MARK CUSHMAN ET AL: "Synthesis and Evaluation of Stilbene and Dihydrostilbene Derivatives as Potential Anticancer Agents That Inhibit Tubulin Polymerization" J. MED. CHEM., Bd. 34, Nr. 8, 1991, Seiten 2579-2588, XP002313141
- DONGHO LEE ET AL: "A Novel Cylooxygenase-Inhibitory Stilbenolignan from the Seeds of Aiphanes aculeata" ORG. LETT., Bd. 3, Nr. 14, 2001, Seiten 2169-2171, XP002313142
- DATABASE BEILSTEIN XP002313149 gefunden im XFIRE Database accession no. 2219534, 2219535 & J. CHEM. SOC. PERKIN TRANS. 1, 1974, Seiten 961-962,
- KSHITIJ THAKKAR ET AL: "Synthesis and Protein-Tyrosine Kinase Inhibitory Activity of Polyhydroxylated Stilbene Analogues of Piceatannol" J. MED. CHEM., Bd. 36, Nr. 20, 1993, Seiten 2950-2955, XP002313143
- DATABASE BEILSTEIN XP002313150 gefunden im XFIRE Database accession no. 2659510 & PHYTOCHEMISTRY, Bd. 10, 1971, Seite 2837,
- MARK CUSHMAN ET AL: "Synthesis and Evaluation of Analogues of (Z)-1-(4-Methoxyphenyl)-2-(3,4,5-trimethox yphenyl)ethene as Potential Cytotoxic and Antimitotic Agents" J. MED. CHEM., Bd. 35, Nr. 12, 1992, Seiten 2293-2306, XP002313144
- DATABASE BEILSTEIN XP002313151 gefunden im XFIRE Database accession no. 9338548 & PHOTOCHEM. PHOTOBIOL., Bd. 76, Nr. 6, 2002, Seiten 596-605,
- MARINELLA ROBERTI ET AL: "Synthesis and Biological Evaluation of Resveratrol and Analogues as Apoptosis-Inducing Agents" J. MED. CHEM., Bd. 46, Nr. 16, 2003, Seiten 3546-3554, XP002313145
- DATABASE BEILSTEIN XP002313152 gefunden im XFIRE Database accession no. 2339832 & PHYTOCHEMISTRY, Bd. 15, 1976, Seite 1057,
- DATABASE BEILSTEIN XP002313153 gefunden im XFIRE Database accession no. 2630218 & HELV. CHIM. ACTA, Bd. 46, 1963, Seiten 1286-1294,
- SANGHEE KIM ET AL: "Design, Synthesis, and Discovery of Novel trans-Stilbene Analogues as Potent and Selective Human Cytochrome P450 1B1 Inhibitors" J. MED. CHEM., Bd. 45, Nr. 1, 2002, Seiten 160-164, XP002313146
- QING-BAI SHE ET AL: "Inhibition of cell transformation by resveratrol and its derivatives: differential effects and mechanisms involved" ONCOGENE, Bd. 22, 2003, Seiten 2143-2150, XP002313147
- MINGFU WANG ET AL: "Evaluation of resveratrol Derivatives as Potential Antioxidants and Identification of a Reaction Product of resveratrol and 2,2-Diphenyl-1-picrylhydrazyl Radical" J. AGRIC. FOOD CHEM., Bd. 47, 1999, Seiten 3974-3977, XP002313148
- DATABASE CAPLUS [Online] CHEMICAL ABSTRACTS SERVICE, COLUMBUS, OHIO, US; DREWES, SIEGFRIED E. ET AL DREWES, SIEGFRIED E. ET AL: "Polyhydroxystilbenes from the heartwood of Schotia brachypetala Polyhydroxystilbenes from the heartwood of Schotia brachypetala" XP002313181 gefunden im STN Database accession no. 1974:449360 & JOURNAL OF THE CHEMICAL SOCIETY, PERKIN TRANSACTIONS 1: ORGANIC AND BIO-ORGANIC CHEMISTRY (1972-1999) , (9), 961-2 CODEN: JCPRB4; ISSN: 0300-922X JOURNAL OF THE CHEMICAL SOCIETY, PERKIN TRANSACTIONS 1: ORGANIC AND BIO-ORGANIC CHEMISTRY (1972-1999), 1974,

## Beschreibung

Die Erfindung betrifft die Verwendung ausgewählter Stilben-Derivate, nämlich
3,5,4'-Trimethoxystilben,
3,3',5,5'-Tetramethoxystilben,
3,4,4',5 -Tetramethoxystilben,
3,3',4,5' -Tetramethoxystilben,
3,3',4,5,5' -Pentamethoxystilben,
3,3',4,4',5,5' -Hexamethoxystilben,
3,3',5,5' -Tetrahydroxystilben,
3,4,4',5 -Tetrahydroxystilben,
3,3',4,5' -Tetrahydroxystilben,
3,3',4,4',5 -Pentahydroxystilben,
3,3',4,5,5' -Pentahydroxystilben,
3,4,4',5,5' -Pentahydroxystilben,
3,3',4',5,5' -Pentahydroxystilben,
3,3',4,4',5,5' -Hexahydroxystilben,
zum Herstellen von Arzneimitteln mit Cyclooxygenase-2 Hemmwirkung.

Arzneimittel, welche die vorgenannten Stilben-Derivate enthalten, sind für alle Zustandsbilder und Erkrankungen, die durch die Verwendung von Cyclooxygenase 2-Hemmem behandelt werden können, geeignet.

### Hintergrund der Erfindung:

Viele natürlich vorkommende Substanzen, wie Flavonoide oder Phenole können das Entstehen einer Reihe von Krankheiten verhindern oder bei der Behandlung verschiedener Erkrankungen, wie zum Beispiel Tumorerkrankungen oder Herz- Kreislauf-Erkrankungen, wirkungsvoll eingesetzt werden. Diese Substanzen können in verschiedenen Pflanzenextrakten, Gewürzmischungen oder Pflanzen, wie Beeren, Trauben, Erdnüssen, oder auch in Wein gefunden werden und wurden beispielsweise bereits in der Indischen oder Chinesischen Medizin eingesetzt.

Resveratrol (3,5,4'-Trihydroxystilben) ist die am Genauesten untersuchte polyphenolische Substanz. Sie wird von Weintrauben gebildet und ist im Wein zu finden. Resveratrol hemmt wirksam das Wachstum von Tumorzellen und wird für das so genannte "französische Paradoxon" (die Tatsache, dass die Wahrscheinlichkeit an koronarer Herzkrankheit zu erkranken, verglichen mit allen anderen Europäischen Staaten in Frankreich um 40% reduziert ist), verantwortlich gemacht. Eine Reihe zellulärer Wirkungen, wie die Hemmung der Cyclooxygenase (COX)-Aktivität (Subbaramaiah K, Chung WJ, Michaluart P, Telang N, Tanabe T, Inoue H, Jang M, Pezzuto JM, Dannenberg AJ. Resveratrol inhibits cyclooxygenase-2 transcription and activity in phorbol ester-treated human mammary epithelial cells, J Biol Chem. 1998 Aug 21;273(34):21875-82.), Hemmung der Ribonukleotid Reduktase Aktivität (Fontecave M, Lepoivre M, Elleingand E, Gerez C, Guittet O. Resveratrol, a remarkable inhibitor of ribonucleotide reductase. FEBS Lett. 1998 Jan 16;421(3):277-9.) oder Induktion von NFkappaB (Tsai SH, Lin-Shiau SY, Lin JK. Suppression of nitric oxide synthase and the down-regulation of the activation of NFkappaB in macrophages by resveratrol. Br J Pharmacol. 1999 Feb;126(3):673-80.) wurden für Resveratrol beschrieben. Eine selektive Hemmung der Cyclooxygenase 2 (COX 2) konnte für Resveratrol nicht gezeigt werden. Dies ist auch nicht der Fall; Resveratrol hemmt beide Isoenzyme (COX 1 und COX 2) mit vergleichbarer Wirksamkeit.

Verschiedene Analoga von Resveratrol werden in der WO 01/21165 A1 als Antitumorsubstanzen genannt. Nicht geoffenbart in der WO 01/21165 A1 ist die Substanz, die Synthese und Verwendung von 3,5,4,4',5,5'-Hexahydroxy, Hexamethoxy- und verwandter Analoga des Resveratrol. Die Hexahydroxy-Verbindung war allerdings unerwartet ein sehr wirksamer Inhibitor des Wachstums von humanen Tumorzellen. Außerdem hat diese Substanz, aber auch die Hexamethoxy-Verbindung, eine selektive Hemmung für COX 2 gezeigt.

Für andere Resveratrol-Analoga beschreiben Ghai et al. (Ghai et al. WO 01/21165 A1, Lu J, Ho CH, Ghai G, Chen KY. Resveratrol analog, 3,4,4',5-tetrahydroxystilbene, differentially induces pro-apoptotic p53/Bax gene expression and inhibits the growth of transformed cells but not their normal counterparts. Carcinogenesis. 2001 Feb;22(2):321-8.) Antitumor-Aktivität, allerdings wird die selektive Hemmung der COX 2 nicht erwähnt.

Zwei Isoenzyme der Cyclooxygenase wurden während der letzten Jahre identifiziert. COX 2 ist die induzierbare Form, die auch gehemmt werden muß, um Entzündungen, Schmerzen etc. zu hemmen. Die Hemmung von COX 1 wird teilweise für die Nebenwirkungen von sogenannten nicht-steroidalen entzündungshemmenden Medikamenten (NSAIDs), wie zum Beispiel Aspirin, verantwortlich gemacht. Deshalb wurden selektive Inhibitoren der COX 2 entwickelt. Dadurch können die Nebenwirkungen der NSAIDs (hauptsächlich gastrointestinale Probleme) minimiert und die Effektivität der Medikamente verbessert werden. Bis jetzt wurden einige wenige hoch selektive Inhibitoren der COX 2 beschrieben. Einige der Medikamente sind zugelassen. Die Indikationen für die Verwendung von COX 2 Inhibitoren sind weiter unten aufgezählt. Sie umfassen inzwischen eine Reihe von Zustandsbildem und Erkrankungen.

Hoch selektive COX 2-Inhibitoren können beispielsweise für die Behandlung folgender Zustandsbilder und Erkrankungen verwendet werden:
Antitumor Wirkung
Behandlung und Prävention von Malignomen
Induktion von Apoptose (programmiertem Zelltod)
Hemmung von NFkappaB
Verwendung in der Kombination mit Strahlentherapie
Verwendung in der Kombination mit anderen Substanzen in der Chemotherapie
Reduktion der Invasivität und des metastatischen Potentials von Tumoren
Fiebersenkende (antipyretische) Wirkung
Hemmung der Gebärmutterkontraktion
Entzündungshemmung
Behandlung von Asthma
Behandlung von Osteoarthritis und rheumatoider Arthritis
Verbessernde Wirkung auf Knochen Reparatur
Prävention und Behandlung von Bindegewebserkrankungen und Knochenerkrankungen einschließlich Osteoporose
Antiöstrogene Effekte (Behandlung zur Tumorprävention und Behandlung von menopausalen und post-menopausalen Beschwerden)
Behandlung von Glaukom
Schmerzreduktion
Reduktion von Ödemen
Antiangiogenetische Wirkung d.h. Wirkung auf Angiogenese (Hemmung der Gefäßbildung)
Hemmung der Plättchenaggregation
Wirkung auf NO Synthase
Prävention von Herz- Kreislauferkrankurigen (Blutgefäßerkrankungen)
Prävention und Behandlung von Herzinfarkt
Prävention und Behandlung von Diabetes und Diabetes Komplikationen
Prävention der ischämisch proliferativen Retinopathie
Prävention von Reperfusionsschäden
Antivirale Aktivität
Antibakterielle Aktivität
Antimykotische Aktivität
Behandlung gegen sonstige Erreger wie: Malariabehandlung
Behandlung von Sichelzellanämie
Behandlung verschiedener Hauterkrankungen (auch lokale topische Verwendung) wie z.B: Psoriasis
Behandlung der Aktinischen Keratose
Prävention und Behandlung der Helicobacter Pylori Gastritis
Prävention und Behandlung von M. Parkinson
Behandlung der Amytopen Lateralsklerose
Behandlung von Multipler Sklerose
Behandlung von M. Alzheimer
Nachstehend werden Beispiele für das Herstellen ausgewählter Stilben-Derivate wiedergegeben, wobei für die Synthese von Methoxystilbenen die Homer-Emmons-Wadsworth-Reaktion angewendet wurde.

Diese Synthese wird an 3',5-Trimethoxystilben gemäß Beispiel 1 erläutert: In einem trockenen Reaktionskolben wurde 10 mmol (2.58 g) Diethyl- (4-methoxybenzyl)phosphonat unter Argon auf 0° C gekühlt. Dann wurden 10 mL trockenes DMF, 20 mmol (1.12 g) Natriummethoxid und 10 mmol (1.661 g) 3,5-Dimethoxybenzaldehyd zugefügt. Das Gemisch wurde bei Zimmertemperatur eine Stunde lang gerührt und dann unter Argon 1,5 Stunden lang auf 100°C erhitzt. Dann wurde die Lösung in ein Becherglas mit 250 mL Eiswasser übergeführt. Der Niederschlag wurde abfiltriert und aus Ethanol (70%) umkristallisiert.
Ausbeute: 1.59 g (59%).

### Beispiel 2:

3,4,4',5-Tetramethoxystilben wurde aus: 10 mmol (2.58 g) Diethyl-(4-methoxybenzyl)-phosphonat, 20 mmol (1.12 g) Natriummethoxid und 10 mmol (1.962 g) 3,4,5-Trimethoxybenzaldehyd auf die oben beschriebene Weise synthetisiert.
Ausbeute: 1.65 g (55%);
Fp=157°C.
¹H-NMR (200 MHz, CDCl₃): δ 7.44 (d, J = 8.8 Hz, 2H), 6.98 (d, J = 16.3 Hz, 1 H), 6.91-6.83 (m, 3H), 6.71 (s, 2H), 3.90 (s, 6H), 3.86 (s, 3H), 3.82 (s, 3H). ¹³C-NMR (50 MHz, CDCl₃): δ 159.2, 153.3, 137.7, 133.3, 129.9, 127.6, 127.5, 126.4,114.1,103.2,60.9,56.0,55.2.
MS m/z 300 (M⁺, 100 %).
Anal (C₁₈H₂₀O₄) C, H.

### Beispiel 3:

3,3' ,5,5' - Tetramethoxystilben wurde wie in Beispiel 1 beschrieben synthetisiert aus: 10 mmol (2.88 g) Diethyl-(3,5-dimethoxybenzyl)phosphonat, 20 mmol (1.12 g) Natriummethoxid und 10 mmol (1.661 g) 3,5-Dimethoxybenzaldehyd.
Ausbeute: 1.56 g (52%).

### Beispiel 4:

3,3',4',5-Tetramethoxystilben wurde wie in Beispiel 1 beschrieben synthetisiert aus: 10 mmol (2.48 g) Diethyl-(3,5-dimethoxybenzyl)phosphonat, 20 mmol (1.12 g) Natriummethoxid und 10 mmol (1.661 g) 3,4-Dimethoxybenzaldehyd.
Ausbeute: 1.65 g (55%).

### Beispiel 5:

3,3',4,5,5'-Pentamethoxystilben wurde wie in Beispiel 1 beschrieben synthetisiert aus: 10 mmol (2.48 g) Diethyl-(3,5-dimethoxybenzyl)phosphonat, 20 mmol (1.12 g) Natriummethoxid und 10 mmol (1.962 g) 3,4,5-Trimethoxybenzaldehyd.
Ausbeute: 1.94 g (59%).

### Beispiel 6:

3,3'4,4',5,5'-Hexamethoxystilben wurde wie in Beispiel 1 beschrieben synthetisiert aus: 10 mmol (3.18 g) Diethyl-(3,4,5-trimethoxybenzyl)phosphonat, 20 mmol (1.12 g) Natriummethoxid and 10 mmol (1.962 g) 3,4,5-Trimethoxybenzaldehyd.
Ausbeute: 1.76 g (49%);
Fp=215°C.
¹H-NMR (200 MHz, CDCl₃): δ 6.94 (s, 2H), 6.74 (s, 4H), 3.92 (s, 12H), 3.87 (s, 6H). ¹³C-NMR (50 MHz, CDCl₃): δ153.3, 137.8, 132.8, 128.0, 103.3, 60.9, 56.0. MS *m*/*z* 360 (M⁺, 100%). Anal. (C₂₀H₂₄O₆) C, H.

### Beispiel 7:

3,4,4',5-Tetrahydroxystilben: In einem trockenen Reaktionskolben wurde 2.5 mmol (0.750 g) 3,4,4',5-Tetramethoxystilben in unter Argon in Methylenchlorid gelöst und auf - 30 °C abgekühlt. Dann wurde tropfenweise 15 mmol (15 mL 1 M-Lösung in Methylenchlorid) Bortribromid-Lösung zugegeben. Die Lösung wurde auf Raumtemperatur erwärmt und 24 Stunden lange gerührt. Die Reaktion wurde durch langsame Zugabe von gesättigter NaHCO₃ Lösung gestoppt. Danach wurde die Lösung für weitere 30 Minuten gerührt und Methylenchlorid wurde abgedampft; die wässrige Phase wurde mit 2N HCl angesäuert. Nach Zugabe von EtOAc wurde das Gemisch extrahiert. Die organische Phase wurde über Na₂SO₄ getrocknet und das Lösungsmittel wurde durch Vakuum entfernt. Die Kristalle wurden aus EtOH/Wasser oder reinem Wasser umkristallisiert.
Ausbeute: 0.335 g (55%);
Fp = 240° C.
¹H-NMR (200 MHz, d₆-DMSO): δ 8.78 (s, 4H), 7.35 (d, *J*= 8.6 Hz, 2H), 6.76-6.72 (m, 4H), 6.47 (s, 2H). ¹³C-NMR (50 MHz, d₆-DMSO): δ 156.7, 146.1, 132.9, 128.5, 128.2, 127.4, 125.9, 125.1, 115.5, 105.2.
MS *m*/*z* 244 (M⁺, 100 %).
Anal. (C₁₄H₁₂O₄) C, H.

### Beispiel 8:

3,3',5,5'-Tetrahydroxystilben wurde aus 2.5 mmol (0.750 g) 3,3',5,5'-Tetramethoxystilben wie in Beispiel 7 beschrieben synthetisiert.
Ausbeute: 0.366 g (60%);
Fp > 320° C.
¹H-NMR (200 MHz, d₆-DMSO): δ 9.24 (s, 4H), 6.84 (s, 2H), 6.41 (d, *J=* 2.0 Hz, 4H), 6.16 (t, *J=* 1.9 Hz, 2H). ¹³C-NMR (50 MHz, d₆-DMSO): δ 158.5, 138.7, 128.4, 128.4, 104.6, 102.2.
MS *m*/*z* 244 (M⁺, 100%).
Anal. (C₁₄H₁₂O₄) C, H.

### Beispiel 9:

3,3',4',5-Tetrahydroxystilben wurde aus 2.5 Mmol (0.750 g) 3,3',4',5-Tetramethoxystilben wie in Beispiel 7 beschrieben, synthetisiert.
Ausbeute: 0.311 g (51 %);
Fp=236°C.
¹H-NMR (200 MHz, d₆-DMSO): δ 8.70 (s, 4H), 6.97 (s, 1H), 6.87- 6.63 (m, 4H), 6.40 (d, 1.9 Hz, 2H), 6.20-6.19 (m, 1H). ¹³C-NMR (50 MHz, d₆-DMSO): δ 158.0, 144.9, 144.8, 138.9, 128.6, 127.8, 125.4, 118.3, 115.3, 112.7, 104.1,101.6.
MS *m*/*z* 244 *(*M⁺*,* 100%).
Anal. (C₁₄H₁₂O₄) C, H.

### Beispiel 10:

3,3',4,5,5'-Pentahydroxystilben wurde aus 2.5 mmol (0.825 g) 3,3',4,5,5'-Pentamethoxystilben wie in Beispiel 7 beschrieben, synthetisiert.
Ausbeute: 0.370 g (57%);
Fp = 252° C.
¹H-NMR (200 MHz, d₆-acetone): δ 8.00 (s, 5H), 6.89 (d, *J=* 16.3 Hz, 1H), 6.76 (d, *J=* 16.3 Hz, 1 H), 6.64 (s, 2H), 6.51 (d, *J*= 2.1 Hz, 2H), 6.28-6.25 (m, 1 H). ¹³C-NMR (50 MHz, d₆-acetone): δ 160.2, 147.4, 141.4, 134.7, 130.5, 130.3, 127.7, 107.3, 106.3, 103.3.
MS *m*/*z* 260 (M⁺, 100%).
Anal (C₁₄H₁₂O₅).

### Beispiel 11:

3,3',4,4',5,5'-Hexahydroxystilben wurde aus 2.5 mmol (0.900 g) 3,3',4,4',5,5'-Hexamethoxystilben wie in Beispiel 1 beschrieben, synthetisiert.
Ausbeute: 0.31 g (45%);
Fp=270°C.
¹H-NMR (200 MHz, d₆-DMSO): δ 8.66 (s, 6H), 6.57 (s, 2H), 6.44 (s, 4H). ¹³C-NMR (50 MHz, d₆-DMSO): δ 146.1, 132.9, 128.1, 125.8, 105.2.
MS *m*/*z* 276 (M⁺, 100%).
Anal. (C₁₄H₁₂O₆) C, H.

Literatur: Chemische Daten von Methoxystilbenen: J. Med. Chem. 45 (1), 1999, p 2671-2686

Die pharmazeutische Wirksamkeit der erfindungsgemäß ausgewählten Stilben-Derivate wurde untersucht:

### 1. Bestimmungsreihe:

### Zell-Kultur:

Die humane Promyelozytenleukämiezellinie HL-60 wurde von der ATCC (American Type Culture Collection, Rockville, MD, USA) gekauft. Die Zellen wurden in RPMI 1640 Medium mit 10% Hitze inaktiviertem fetalem Kälberserum (FCS) (GIBCO, Grand Island Biological Co., Grand Island, NY, USA) und mit 1 % Penizillin/Streptomycin in angefeuchteter Atmosphäre bei 5% CO₂ gezüchtet.

Die Zellzahlen wurden mittels eines Mikrozellcounters CC-108 (Sysmex, Kobe, Japan) bestimmt. Für die Versuche wurden Zellen in der logarithmischen Wachstumsphase verwendet.

### Wachstumshemmungsassay:

Logarithmisch wachsende HL-60 Zellen wurden in einer Dichte von 0.1x10⁶ Zellen/ml in Gewebekulturflaschen angesetzt und mit verschiedenen Konzentrationen der zu untersuchenden Stilben-Derivate inkubiert. Nach 72 Stunden wurden die Zellen mittels des Mikrozellcounters gezählt. Die Viabilität der Zellen wurde mittels Trypanblau Färbung bestimmt. Als Resultate wurden als Anzahl viabler Zellen berechnet.

### COX (humaner) Inhibitor Screening Assay:

Es wurde ein Immunoassay der Firma IBL Produkte, Hamburg, Deutschland für die Bestimmung der COX 1 und COX 2 Aktivitäten verwendet. Der Assay bestimmt quantitativ die Prostaglandine F, E und D sowie Thromboxan B artige Prostaglandine, die durch die Cyclooxygenase Reaktion gebildet werden. COX 1 und COX 2 wurden als so- genannte IC₅₀, 50 % Enzym Hemmung, d.h. die Substanzkonzentration, welche 50 % des gemessenen Isoenzymes hemmt, angegeben.

**Tabelle 1:**

| Hemmende Wirkung von Stilbenen und von Resveratrol auf COX 1 und COX 2 Aktivität: | | | |
|---|---|---|---|
| Stoff | IC₅₀ (µM) | | COX 2/ COX 1 |
| | COX 1 | COX 2 | Verhältnis |
| 3,3',4,5,5'-Pentamethoxystilben | 10 | 0,6 | 0,06 |
| 3,3',4,4',5,5'-Hexamethoxystilben | 10 | 0,5 | 0,05 |
| 3,4,4',5-Tetrahydroxystilben | 5 | 0,01 | 0,002 |
| 3,3',5,5'-Tetrahydroxystilben | 0,01 | < 0,001 | <0,1 |
| 3,3',4',5-Tetrahydroxystilben | 5 | 0,005 | 0,001 |
| | | | |
| 3,3',4',5,5'-Pentahydroxystilben | 0,01 | 0,005 | 0,5 |
| 3,3',4,4',5,5'-Hexahydroxystilben | 0,5 | 0,001 | 0,002 |
| 3,4',5-Trihydroxystilben (Resveratrol) | 0,5 | 0,5 | 1 |

Wie aus der Tabelle 1 ersichtlich ist, sind nur die erfindungsgemäß ausgewählten Verbindungen, nicht aber Resveratrol selbst COX 2 selektiv (d.h. sie zeigen eine wesentlich effektivere Wirkung auf COX 2 als auf COX 1).

**Tabelle 2:**

| Hemmende Wirkung erfindungsgemäßer Verbindungen auf das Wachstum von HL-60 humane Promyelocyten Leukämie-Zellen: | |
|---|---|
| Stoff | IC₅₀ (µM) |
| 3,3',4,5,5'-Pentamethoxystilben | 25 |
| 3,3',4,4',5,5'-Hexamethoxystilben | >100 |
| 3,4,4',5-Tetrahydroxystilben | 9 |
| 3,3',5,5'-Tetrahydroxystilben | 12,5 |
| 3,3',4',5-Tetrahydroxystilben | 9 |
| 3,3',4',5,5'-Pentahydroxystilben | 10 |
| 3,3',4,4',5,5'-Hexahydroxystilben | 4 |
| 3,4',5-Trihydroxystilbene (Resveratrol) | 12 |

### Bestimmungsreihe:

### Chemikalien

3, 4, 5, 3`, 4', 5'- Hexahydroxystilben wurde gemäß Beispiel 11 synthetisiert und verwendet. Resveratrol stammt von Sigma-Aldrich GmbH, Vienna, Austria. Beide Substanzen wurden in DMSO verdünnt.

### Zell Kultur

Die humane Promyelozytenleukämiezellinie HL-60 wurde von der ATCC(American Type Culture Collection, Rockville, MD, USA) gekauft. Die Zellen wurden in RPMI 1640 Medium mit 10% Hitze inaktiviertem fetalem Kälberserum (FCS) (GIBCO, Grand Island Biological Co., Grand Island, NY, USA) und mit 1 % Penizillin/Streptomycin in angefeuchteter Atmosphäre bei 5% CO₂ gezüchtet.

Die Zellzahlen wurden mittels eines Mikrozellcounters CC-108 (Sysmex, Kobe, Japan) bestimmt. Für die Versuche wurden Zellen in der logarithmischen Wachstumsphase verwendet.

### Wachstumshemmungsassay

Logarithmisch wachsende HL-60 Zellen wurden in einer Dichte von 0.1x10⁶ Zellen/ml in Gewebekulturflaschen angesetzt und mit verschiedenen Konzentrationen der Resveratrol Analoga inkubiert. Nach 72 Stunden wurden die Zellen mittels des Mikrozellcounters gezählt. Die Viabilität der Zellen wurde mittels Trypanblau Färbung bestimmt. Als Resultate wurden als Anzahl viabler Zellen berechnet.

Analyse der intrazellulären dNTP (Deoxynukleosidtriphosphat) Pools mittels Hochdruckflüssigkeitschromatographie (HPLC)

Die Analyse der intrazellulären dNTP Konzentrationen erfolgte nach der Methode von Garrett und Santi (Garrett C, Santi DV. A rapid and sensitive high pressure liquid chromatography assay for deoxyribonucleoside triphosphates in cell extracts. Anal Biochem. 1979 Nov 1;99(2):268-73.)

### Höchst Propidium Jodid Doppelfärbung

HL-60 Zellen (0.1 x 10⁶/ml) wurden in 25 cm² Flaschen gesät und 24 Stunden lange mit Hexahydroxystilben bzw. Resveratrol inkubiert. Dann wurden die Zellen mit Hoechst 33258 (HO, Sigma, St. Louis, MO, USA) und Propidium Iodid (PI, Sigma, St. Louis, MO, USA) (5 µg/ml und 2 µg/ml) eine Stunde lange behandelt. Danach wurden die Zellen mittels eines Fluoreszenz Mikroskops fotografiert und die Zellen wurden morphologisch in früh apoptoisch, spät apoptotisch und nekrotische Zellen unterteilt.

### Bestimmung der Zell Zyklus Verteilung

HL-60 Zellen wurden in der Gegenwart von Hexahydroxystilben inkubiert. Nach 24 Stunden wurden die Zellen gewaschen, zentrifugiert und in Alkohol fixiert. Danach mit Propidium Iodid gefärbt und mittels FACS Analyse untersucht. Danach wurde die Zell Zyklus Phasen Verteilung berechnet.

In unbehandelten Zellen waren 41.9% in GO-G1, 43.8% in S und 14.3% in G2-M Phase des Zell Zyklus.

### Ergebnisse der 2. Bestimmungsreihe:

In der folgenden Beschreibung wird auf die Fig. 1 bis 5 Bezug genommen. Diese zeigen:

### Legende zu den Abbildungen

Fig. 1: Zytotoxische Wirkungen von Resveratrol und Hexahydroxystilben in humanen HL-60 Leukämie Zellen
Fig. 2: Wirkung von Vitamin C und Hexahydroxystilben auf das Wachstum von HL-60 Zellen
Fig. 3: Wirkung von Hexahydroxystilben auf intrazelluläre dNTP Spiegel von HL-60 Zellen.
Fig. 4: Apoptose Induktion durch Resveratrol und Hexahydroxystilben in HL-60 Zellen
Fig. 5: Zell Zyklus Phasen Verteilung von HL-60 Zellen nach Behandlung mit Hexahydroxystilben

### Wachstumshemmungsassay

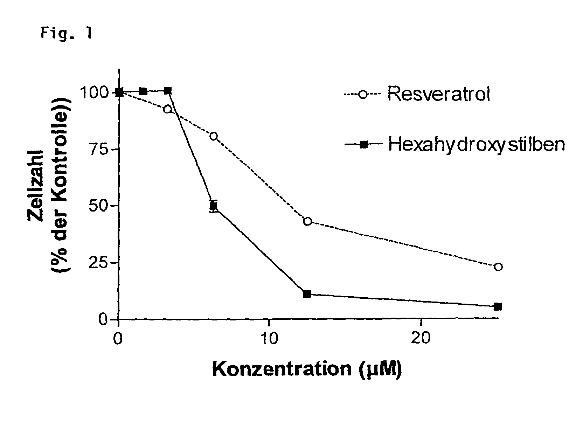

Die wachstumshemmende Wirkung von Resveratrol bzw. Hexahydroxystilben ("M8") auf HL-60 Zellen ist in Fig. 1 dargestellt. Nach 72 Stunden Inkubation hat Resveratrol eine IC50 (50 % Hemmung des Zellwachstums) von 12 µM bewirkt, während Hexahydroxystilben die Zellen mit einem IC50 Wert von 6,25 µM an ihrem Wachstum gehemmt hat.

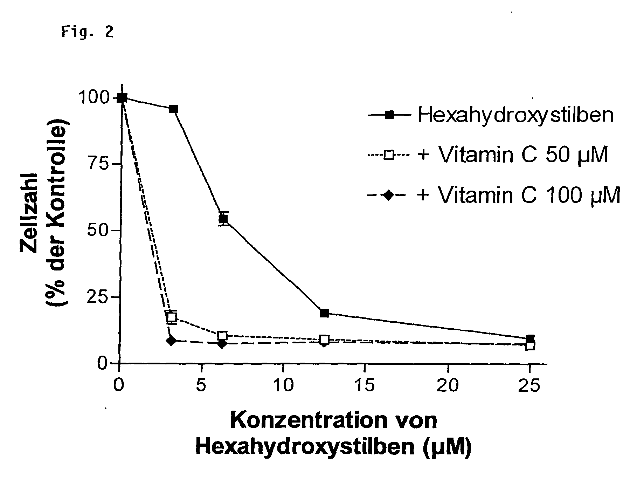

Durch Zugabe von 50 bzw. 100 µM Vitamin C konnte das wachstumshemmende Potential der beiden Substanzen, wie in Fig. 2 dargestellt, noch weiter verstärkt werden. Für Hexahydroxystilben ("M8") z.B. auf einen IC50 Wert von 2 µM. Vitamin C selbst hat in den verwendeten Konzentrationen keine Wirkung auf das Zellwachstum gezeigt.

### Analyse der intrazellulärene dNTP (Deoxynucleosidtriphosphat) Pools

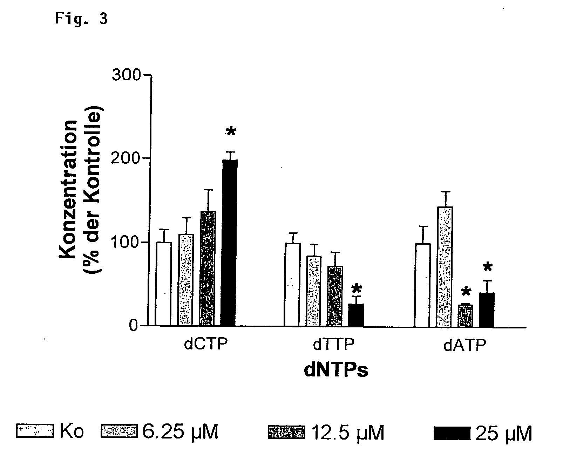

Untersucht wurde die Wirkung von Hexahydroxystilben ("M8") auf die intrazellulären dNTP Konzentrationen in HL-60 Zellen. Die Untersuchung wurde wie oben beschrieben durchgeführt. Die Zellen wurden mit 6,25, 12,5 und 25 µM Hexahydroxystilben inkubiert und dann wurden die dNTP Pools bestimmt. Die dCTP Pools erhöhten sich auf 110, 137 und 199 % der Kontroll- Werte, während die dTTP Pools auf 84, 72 und 27 % der Ausgangswerte sanken. Die dATP Konzentrationen sanken bei Behandlung mit 12,5 und 25 µM Hexahydroxystilben auf 27 und 41 % der Ausgangswerte. Besonders eindrucksvoll war die Depletion der dATP Pools, im Falle von HT-29 humanen Kolon Tumor Zellen, dort sanken die dATP Werte bereits nach Behandlung mit 4 µM der Substanz auf durchschnittlich 1,5 % der Ausgangswerte. Es gibt kaum eine antivirale bzw. in der Antitumorbehandlung eingesetzte Substanz, welche solche eindrucksvollen Wirkungen zeigt. Durch diese eindrucksvolle Inbalance der dNTP Pools, welche Prekursoren der DNA Synthese sind, kann auch die Wirkung der Substanz erklärt werden. Die Ergebnisse des Experimentes mit HL-60 Zellen sind in Fig. 3 dargestellt.

### Induktion von Apoptose durch Resveratrol bzw. Hexahydroxystilben

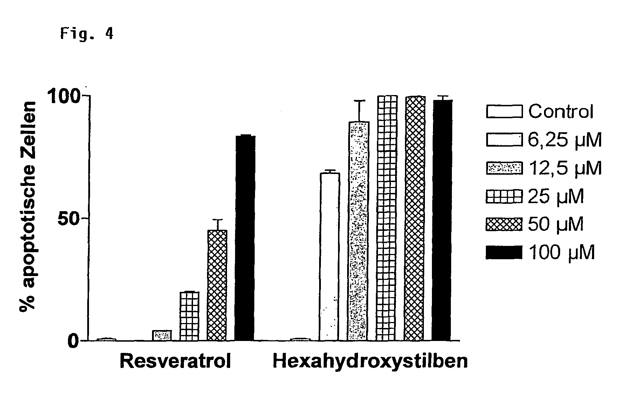

Es ist bekannt, daß Resveratrol in verschiedenen Tumorzellen Apoptose induziert. Wir haben die apoptotische Wirkung von Resveratrol mit der von Hexahydroxystilben ("M8") verglichen. HL-60 Leukämie Zellen wurden 24 Stunden lange mit verschiedenen Konzentrationen von Resveratrol bzw. Hexahydroxystilben ("M8") behandelt; dann wurde die Anzahl von apoptotischen Zellen mittels Höchst/Propidium Jodid Doppelfärbung bestimmt. Die Resultate sind in Fig. 4 dargestellt. Hexahydroxystilben ("M8") hat, wie aus der Abbildung ersichtlich bei signifikant niedrigeren Konzentration als Resveratrol in diesen Zellen Apoptose induziert. Behandlung mit 6.25 µM Hexahydroxystilben ("M8") führte zum Beispiel in 68.5% der Zellen zur Induktion von Apoptose.

Wirkung von Hexahydroxystilben auf die Zell Zyklus Verteilung von HL-60 Leukämie Zellen

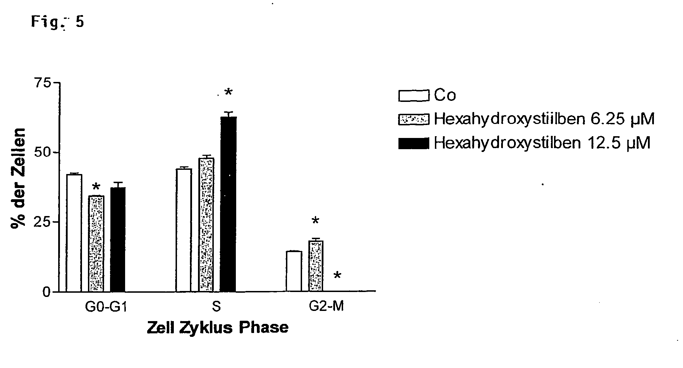

Wie aus Fig. 5 ersichtlich, hat Hexahydroxystilben ("M8") signifikanten Einfluß auf die Zell Zyklus Verteilung von HL-60 Zellen. Behandlung mit Hexahydroxystilben hat die Zellen in S Phase arretiert und so eine Hemmung des Zellwachstums bewirkt. Es kam in der Folge nach Inkubation mit Hexahydroxystilben zu einer Depletion von Zellen in G2-M Phase des Zell Zyklus.

In der folgenden Tabelle 3 sind die Wirksamkeiten von Resveratrol und ausgewählter erfindungsgemäßer Verbindungen wiedergegeben:

Wachstumstrennung (72h) von Stilben-Derivaten in HL-60 und klonogenen Testreihen (7d) in Prostata-Krebs-Zelllinien:

| Verbindung | **Kode** | **HL-60** | **PC-3** | **LNCAP*** | **DU-145** |
|---|---|---|---|---|---|
| **Resveratrol** | | 12 µM | 16 µM | 5 µM | 10 µM |
| **3,5,3',5'-tetramethoxy** | M1 | über 100 µM | 35 µM | 21 µM | 25 µM |
| **3,4,5,3'5'-pentamethoxy** | M2 | 25 µM | 21 µM | über 100 µM | 68 µM |
| **3,4,5,3',4',5'-hexamethoxy** | M3 | über 100 µM | 5,5 µM | 37 µM | 12,5 µM |
| **3,4,5,4'-tetramethoxy** | M4 | 20 µM | 3 µM | 0,4 µM | 0,4 µM |
| **3,5,4'-trimethoxy** | M5 | 5 µM | 3 µM | 6,25 µM | 6 µM |
| **3,4,3',5'-tetramethoxy** | M5A | 25 µM | 35 µM | 6,25 µM | 30 µM |
| **3,5,3',5'-tetrahydroxy** | M6 | 12,5 µM | 16 µM | 84 µM | 17 µM |
| **3,4,5,3'5'-pentahydroxy** | M7 | 10 µM | 5 µM | 7,5 µM | 23 µM |
| **3,4,5,3',4',5'-hexahydroxy** | M8 | 4 µM | 2,5 µM | 3,4 µM | 25 µM |
| **3,4,5,4'-tetrahydroxy** | M9 | 9 µM | 8 µM | 9,5 µM | 30 µM |
| **3,4,3',5'-tetrahydroxy** | M10 | 9 µM | 18 µM | 33 µM | 58 µM |

| | | | | | |
|---|---|---|---|---|---|
| * LNCaP-Zellen sind zu 5-alpha-dihydrotestosterone responsiv. ** jeweils " -stilben" | | | | | |

Zusammenfassend kann ein Ausführungsbeispiel der Erfindung wie fölgt dargestellt werden:

Beschrieben werden Stilbenderivate der alllgemeinen Formel 1

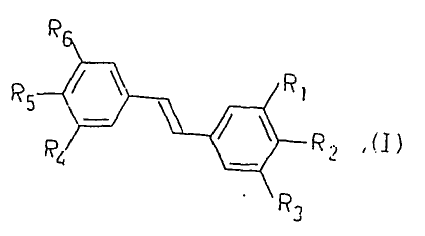

In dieser haben wenigstens vier der Substituenten R₁ bis R₆ eine andere Bedeutung als Wasserstoff. Die Substituenten sind wirksame Radikalfänger, Antitumorwirkstoffe und selektive Cyclooxygenase-2-Inhibitoren.

## Patentansprüche

1. Verwendung ausgewählter Stilben-Derivate, nämlich
3,5,4'-Trimethoxystilben,
3,3',5,5'-Tetramethoxystilben,
3,4,4',5 -Tetramethoxystilben,
3,3',4,5' -Tetramethoxystilben,
3,3',4,5,5' -Pentamethoxystilben,
3,3',4,4',5,5' -Hexamethoxystilben,
3,3',5,5' -Tetrahydroxystilben,
3,4,4',5 -Tetrahydroxystilben,
3,3',4,5' -Tetrahydroxystilben,
3,3',4,4',5 -Pentahydroxystilben,
3,3',4,5,5' -Pentahydroxystilben,
3,4,4',5,5' -Pentahydroxystilben,
3,3',4',5,5' -Pentahydroxystilben,
3,3',4;4',5,5' -Hexahydroxystilben,
zum Herstellen von Arzneimitteln mit Cyclooxygenase-2 Hemmwirkung.

## Claims

1. Use of selected stilbene derivatives, namely
3,5,4'-Trimethoxystilbene,
3,3',5,5'-Tetramethoxystilbene,
3,4,4',5-Tetramethoxystilbene,
3,3',4,5'-Tetramethoxystilbene,
3,3',4,5,5' -Pentamethoxystilbene,
3,3',4,4',5,5'-Hexamethoxystilbene,
3,3',5,5'-Tetrahydroxystilbene,
3,4,4',5-Tetrahydroxystilbene,
3,3',4,5'-Tetrahydroxystilbene,
3,3',4,4',5 -Pentahydroxystilbene,
3,3',4,5,5'-Pentahydroxystilbene,
3,4,4',5,5' -Pentahydroxystilbene,
3,3',4',5,5'-Pentahydroxystilbene,
3,3',4,4',5,5'-Hexahydroxystilbene,
for the production of drugs with cyclooxygenase-2 inhibiting action.

## Revendications

1. Utilisation de dérivés de stilbène sélectionnés, plus précisément du
3,5,4'-triméthoxystilbène,
3,3',5,5'-tétraméthoxystilbène
3,4,4',5-tétraméthoxystilbène
3,3',4,5'-tétraméthoxystilbène
3,3',4,5,5'-pentaméthoxystilbène
3,3',4,4',5,5'-hexaméthoxystilbène
3,3',5,5'-tétrahydroxystilbène
3,4,4',5-tétrahydroxystilbène
3,3',4,5'-tétrahydroxystilbène
3,3',4,4',5-pentahydroxystilbène
3,3',4,5,5'-pentahydroxystilbène
3,4,4',5,5'-pentahydroxystilbène
3,3',4',5,5'-pentahydroxystilbène
3,3',4,4',5,5'-hexahydroxystilbène,
pour la préparation de médicaments possédant un effet inhibiteur de la cyclooxygénase-2.
